(19) Europäisches Patentamt
European Patent Office
Office européen des brevets

(11) EP 4 721 754 A1

(12) EUROPEAN PATENT APPLICATION
published in accordance with Art. 153(4) EPC

(43) Date of publication:
**08.04.2026 Bulletin 2026/15**

(21) Application number: **24814430.5**

(22) Date of filing: **28.05.2024**

(51) International Patent Classification (IPC):
***A61K 38/26*** *(2006.01)* ***A61K 47/54*** *(2017.01)*
***A61K 9/00*** *(2006.01)* ***A61P 3/10*** *(2006.01)*
***C07K 14/62*** *(2006.01)*

(52) Cooperative Patent Classification (CPC):
**A61K 9/00; A61K 38/26; A61K 47/54; A61P 3/10; C07K 14/62**

(86) International application number:
**PCT/CN2024/095763**

(87) International publication number:
**WO 2024/245233 (05.12.2024 Gazette 2024/49)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.05.2023 CN 202310619176**

(71) Applicants:
- **Hangzhou Sciwind Biosciences Co., Ltd.**
  **Hangzhou, Zhejiang 310015 (CN)**
- **Sciwind Biosciences (Beijing) Co., Ltd.**
  **Beijing 100176 (CN)**

(72) Inventors:
- **LI, Yan**
  **Beijing 100176 (CN)**
- **HAO, Sujuan**
  **Beijing 100176 (CN)**
- **LI, Zhaoying**
  **Beijing 100176 (CN)**
- **PAN, Hai**
  **Beijing 100176 (CN)**
- **ZOU, Haixia**
  **Beijing 100176 (CN)**
- **WU, Xinle**
  **Beijing 100176 (CN)**

(74) Representative: **Snaith, James Michael et al**
**Kilburn & Strode LLP**
**Lacon London**
**84 Theobalds Road**
**London WC1X 8NL (GB)**

Remarks:
The complete document including Reference Table(s) and the Sequence Listing(s) can be downloaded from the EPO website

# (54) POLYPEPTIDE DERIVATIVE FOR ORAL DELIVERY

(57) Provided herein are use of a fatty acid-containing side chain in improving activity, activity selectivity, and oral availability of a polypeptide derivative, a method for achieving the use, and a polypeptide derivative modified by the side chain.

EP 4 721 754 A1

## Description

## TECHNICAL FIELD

The present application relates to the technical field of biomedicine or biopharmaceuticals, specifically, a polypeptide or a derivative thereof for oral delivery, a composition of the polypeptide or the derivative thereof for oral delivery, and use of them for treating and/or preventing metabolism associated diseases.

## BACKGROUND

Polypeptides/proteins are not only indispensable elements as an essential cellular component in an organism, but also play important roles in maintaining normal physiological activities of the organism as major participants of intercellular or inter-tissue signaling. Since bovine insulin and porcine insulin were used for treatment of diabetes for the first time in the early 2000s, more and more polypeptides/proteins have been produced and applied in the field of human pharmaceuticals as modern technologies of molecular biology and organic chemistry advance. Polypeptide/protein-based drugs have distinct advantages in treatment of diseases including cancer, diabetes, cardiocerebrovascular diseases, autoimmune diseases, and metabolic disorders over their counterparts of other types due to their high specificity for targets as well as desirable safety and tolerance. However, a large proportion of polypeptide/protein-based drugs with therapeutic activity have only modest oral bioavailability due to limitations imposed by the mechanism of absorption of polypeptides/proteins in the human body, and thus need to be administered by injections or other routes for treating diseases, which hinders the development of polypeptide/protein-based drugs and brings about additional pain and inconvenience for patients receiving the treatment.

Many attempts have been made to enhance the effect of orally administered polypeptide-based drugs in order to fix that deficiency in oral polypeptides. For example, Fiona McCartney et al. (Journal of Controlled Release, Volume 310, 28 September 2019, Pages 115-126) disclosed Labrasol® ALF (Labrasol®), a non-ionic surfactant excipient for use in enhancing bioavailability of insulin in the intestine; P. Uhl et al. (Nanomedicine: Nanotechnology, Biology and Medicine, Volume 24, February 2020, 102132) discovered that coating of PLA nanoparticles with cyclic, arginine-rich cell penetrating peptides enables oral delivery of liraglutide; and Vivek Gupta et al. (Journal of Controlled Release, Volume 172, Issue 3, 28 December 2013, Pages 753-762) disclosed preparation of mucoadhesive devices by compressing a polymeric matrix containing carbopol, pectin and sodium carboxymethylcellulose (1:1:2), and demonstrated its effect of improving salmon calcitonin (sCT) delivery *in vivo.*

However, current polypeptide-based drugs still have problems such as poor oral efficacy, low bioavailability, unpredictable delivery effects of corresponding oral administration regimens for the polypeptide molecules, and poor versatility.

## SUMMARY OF THE INVENTION

Accordingly, the present application provides for a polypeptide derivative with increased activity and stability as well as an improved effect and bioavailability for oral delivery, which is capable of effectively treat and/or prevent metabolism associated diseases. Specifically, the present application is directed to a technical solution comprising the following embodiments:

1. Use of a fatty acid-containing side chain in improving activity selectivity of a polypeptide derivative, wherein the polypeptide derivative is formed by modification of a polypeptide molecule comprising the fatty acid-containing side chain, wherein the fatty acid-containing side chain is in the form of Z1+Z2+Z3, wherein Z1 is a C16-C22 fatty diacid;

   Z2 is selected from any one of yGlu, αGlu, βAsp, αAsp, Inp, and Trx, or is absent; and
   Z3 is n AEEA(s), wherein $n \geq 0$.

2. Use of a fatty acid-containing side chain in improving activity of a polypeptide derivative, wherein the polypeptide derivative is formed by modification of a polypeptide molecule comprising the fatty acid-containing side chain, wherein the fatty acid-containing side chain is in the form of Z1+Z2+Z3, wherein Z1 is a C16-C22 fatty diacid;

   Z2 is selected from any one of yGlu, αGlu, βAsp, αAsp, Inp, and Trx, or is absent; and
   Z3 is n AEEA(s), wherein $n \geq 0$.

3. Use of a fatty acid-containing side chain in improving oral bioavailability of a polypeptide derivative, wherein the polypeptide derivative is formed by modification of a polypeptide molecule comprising the fatty acid-containing side

chain, wherein the fatty acid-containing side chain is in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein n≥0.

4. A method of improving activity selectivity of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein n≥0.

5. A method of improving activity of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein n≥0.

6. A method of improving oral bioavailability of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein n≥0.

7. The use of any one of embodiments 1-3 or the method of any one of embodiments 4-6, wherein Z1 is a C16, C17, C18, C19 or C20 fatty diacid.
8. The use or the method of any one of embodiments 1-7, wherein Z2 is selected from any one of yGlu, βAsp, γGlu, Inp, and Trx, preferably βAsp or yGlu.
9. The use or the method of any one of embodiments 1-8, wherein n is greater than 0, preferably 1-10, more preferably 2-8, and further preferably, n=2.
10. The use or the method of any one of embodiments 1-9, wherein Z1 is a C16, C18 or C20 fatty diacid.
11. The use or the method of any one of embodiments 1-10, wherein Z2 is Inp or Trx; and n is greater than 0.
12. The use or the method of any one of embodiments 1-11, wherein Z1 is a C16, C17, C18, C19 or C20 fatty diacid; Z2 is γGlu; and n=2.
13. The use or the method of any one of embodiments 1-12, wherein Z1, Z2, and Z3 are linked via amide linkages.
14. The use or the method of any one of embodiments 1-13, wherein the modification of the side chain is on 1-10 amino acids, preferably 1-3 amino acids, and more preferably 1 amino acid, of the polypeptide.
15. The use or the method of any one of embodiments 1-14, wherein the modification of the side chain is at an amino acid at the N terminus or any middle position in the sequence of the polypeptide, wherein the amino acid is preferably selected from glutamic acid, aspartic acid, lysine, glutamine, or asparagine, and is further preferably lysine.
16. The use or the method of any one of embodiments 1-15, wherein the polypeptide is an agonistic or antagonistic, natural or artificially synthetic polypeptide molecule, preferably selected from a GLP-1 receptor agonist, a GIP receptor antagonist, an insulin receptor agonist, a heparin receptor agonist, somatotrophin, an interferon receptor agonist, an interleukin receptor agonist, a follicle-stimulating hormone receptor agonist, a gonadotropin receptor agonist, an erythropoietin receptor agonist, a Peptide YY (PYY) receptor agonist, an oxygenation modulator receptor agonist, a glucagon-like peptide-2 (GLP-2) receptor agonist, a calcitonin receptor agonist, a parathyroid hormone (PTH) receptor agonist, a ghrelin receptor agonist, an endocannabinoid receptor agonist, a leptin receptor agonist, a serotonin receptor agonist, a fibroblast growth factor 21 (FGF21) receptor agonist, a cholecystokinin (CCK) receptor agonist, an oxyntomodulin receptor agonist, or a glucagon receptor agonist, preferably an amylin receptor agonist, and further preferably an amylin or calcitonin analog.
17. The use or the method of any one of embodiments 1-16, wherein the polypeptide comprises an amino acid sequence of ASQLS TAVLG RLSDE LHRLQ DYPRT DVGSG SP-$NH_2$, KCNTATCATQ RLADFLRHSS NNLKPILPPT NVGSNT-trans-Hyp-$NH_2$, or KCNTATCATQ RLAEFLRHSS NNFGPILPPT NVGSNTP-$NH_2$, and wherein

preferably, the polypeptide derivative is selected from any one or more of N1 or N2 or M1-M30, or
preferably, the polypeptide derivative is selected from any one or more of N1, N2, N3, or M1-M30.

18. The use of embodiment 1 or any one of embodiments 7-17 or the method of embodiment 4 or any one of embodiments 7-17, wherein the improving the activity selectivity of the polypeptide derivative is improving the activity of the polypeptide derivative against an amylin receptor preferentially over a calcitonin receptor, preferably is improving a ratio of the activity of the polypeptide derivative against an amylin receptor to that against a calcitonin receptor, and further preferably comprises improving the activity of the polypeptide derivative against an amylin receptor.

19. The use of embodiment 1 or any one of embodiments 7-18 or the method of embodiment 4 or any one of embodiments 7-18, wherein the improving the activity selectivity of the polypeptide derivative is improving the ratio of the activity against an amylin receptor to that against a calcitonin receptor in a cell by more than 1.5 folds, preferably more than 2 folds, further preferably more than 5 folds, and still further preferably more than 10 folds.

20. The use or the method of embodiment 18 or 19, wherein the activity is agonistic activity.

21. The use of embodiment 1 or any one of embodiments 7-20 or the method of embodiment 4 or any one of embodiments 7-20, wherein the polypeptide derivative is an amylin receptor agonist, preferably is an amylin or calcitonin derivative, and more preferably comprises an amino acid sequence of ASQLS TAVLG RLSDE LHRLQ DYPRT DVGSG SP-$NH_2$, or further preferably comprises an amino acid sequence of KCNTATCATQ RLADFLRHSS NNLKPILPPT NVGSNT-trans-Hyp-$NH_2$.

22. The use of embodiment 2 or any one of embodiments 7-17 or the method of embodiment 5 or any one of embodiments 7-17, wherein the activity of the polypeptide derivative is activity in losing weight and/or reducing food intake.

23. The use of embodiment 2 or any one of embodiments 7-17 or the method of embodiment 5 or any one of embodiments 7-17, wherein the improving the activity of the polypeptide derivative is improving the activity of the polypeptide derivative against an amylin receptor and a calcitonin receptor in a cell.

24. The use of embodiment 2 or any one of embodiments 7-17 and 22-23 or the method of embodiment 5 or any one of embodiments 7-17 and 22-23, wherein the polypeptide derivative is an amylin receptor agonist, preferably is an amylin or calcitonin derivative, and more preferably comprises an amino acid sequence of KCNTATCATQ RLADFLRHSS NNLKPILPPT NVGSNT-trans-Hyp-$NH_2$ or KCNTATCATQ RLAEFLRHSS NNFGPILPPT NVGSNTP-$NH_2$.

25. The use of embodiment 3 or any one of embodiments 7-17 or the method of embodiment 6 or any one of embodiments 7-17, wherein the polypeptide derivative with the fatty acid-containing side chain is co-administered with one or more oral delivery agents, preferably a salt of N-[8-(2-hydroxybenzoyl)amino]caprylic acid (NAC), and more preferably PNAC.

26. The use of embodiment 3 or any one of embodiments 7-17 or the method of embodiment 6 or any one of embodiments 7-17, wherein the improving the oral bioavailability of the polypeptide derivative is achieving higher oral bioavailability than a polypeptide molecule of Cagrilintide.

27. The use of embodiment 3 or any one of embodiments 7-17 or the method of embodiment 6 or any one of embodiments 7-17, wherein the polypeptide derivative is an amylin receptor agonist, preferably is an amylin or calcitonin derivative, and more preferably comprises an amino acid sequence of KCNTATCATQ RLADFLRHSS NNLKPILPPT NVGSNT-trans-Hyp-$NH_2$ or KCNTATCATQ RLAEFLRHSS NNFGPILPPT NVGSNTP-$NH_2$.

28. A derivative of a polypeptide, comprising the polypeptide and a side chain linked thereto in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein n≥0.

29. The derivative of embodiment 28, wherein Z1 is a C16, C17, C18, C19 or C20 fatty diacid.

30. The derivative of embodiment 28 or 29, wherein Z2 is selected from any one of γGlu, Inp, and Trx, or is absent.

31. The derivative of any one of embodiments 28-30, wherein n is greater than 0.

32. The derivative of any one of embodiments 28-31, wherein Z2 is Inp or Trx; and n is greater than 0.

33. The derivative of any one of embodiments 28-32, wherein Z2 is absent; and n is greater than 0.

34. The derivative of any one of embodiments 28-33, wherein Z2 is yGlu; and n=2.

35. The derivative of any one of embodiments 28-34, wherein the polypeptide is an amylin receptor agonist; preferably, the derivative of the polypeptide is an amylin or calcitonin derivative; and more preferably, the polypeptide comprises an amino acid sequence of ASQLS TAVLG RLSDE LHRLQ DYPRT DVGSG SP-$NH_2$, KCNTATCATQ RLADFLRHSS NNLKPILPPT NVGSNT-trans-Hyp-$NH_2$, or KCNTATCATQ RLAEFLRHSS NNFGPILPPT NVGSNTP-$NH_2$, and wherein

preferably, the derivative of the polypeptide is selected from any one or more of N1 or N2 or M1-M30, or
preferably, the derivative of the polypeptide is selected from any one or more of N1, N2, N3, or M1-M30.

36. A pharmaceutical composition comprising the derivative of any one of embodiments 28-35 or a pharmaceutically acceptable salt thereof, and a pharmaceutically acceptable excipient, wherein preferably, the composition comprises one or more oral delivery agents, preferably a salt of N-[8-(2-hydroxybenzoyl)amino]caprylic acid (NAC), and further preferably PNAC.
37. Use of the derivative of any one of embodiments 28-35 or a pharmaceutically acceptable salt thereof or the composition of embodiment 36 in the manufacture of a polypeptide-based medicament; wherein preferably, the polypeptide-based medicament is used for preventing and/or treating overweight and/or obesity and/or type I or type II diabetes and/or osteoporosis and/or neuropathic pain.
38. The use of embodiment 37, wherein the polypeptide-based medicament is an oral medicament; and preferably, the oral medicament comprises one or more oral delivery agents, preferably a salt of N-[8-(2-hydroxybenzoyl)amino] caprylic acid (NAC), and further preferably PNAC.
39. Use of the derivative of any one of embodiments 28-35 or a pharmaceutically acceptable salt thereof or the pharmaceutical composition of embodiment 36 in reducing food intake.
40. A method of preventing and/or treating overweight and/or obesity and/or type I or type II diabetes and/or osteoporosis and/or neuropathic pain, comprising administering to a subject a prophylactically or therapeutically effective amount of the derivative of any one of embodiments 28-35 or a pharmaceutically acceptable salt thereof or the pharmaceutical composition of embodiment 36.
41. A method of reducing food intake, comprising administering to a subject an effective amount of the derivative of any one of embodiments 28-35 or a pharmaceutically acceptable salt thereof or the pharmaceutical composition of embodiment 36.

## TECHNICAL EFFECT

The polypeptide derivatives of the present application have high activity in cells, strong potency or effectiveness on amylin and calcitonin receptors, and stable characteristics and properties including concentration and purity with little change after storage for a long period of time.

The polypeptide derivatives of the present application produce prominent effects on losing weight and reducing food intake and attain enhanced efficacy and bioavailability as oral drugs.

## BRIEF DESCRIPTION OF THE DRAWINGS

The drawings are intended for a better understanding rather than any undue limitation of the present application, in which:

FIG. 1 is a trend chart showing the effect of the polypeptide derivatives on weight change of rats;
FIG. 2 is a trend chart showing the effect of the polypeptide derivatives on accumulated food intake of rats;
FIG. 3 is a trend chart showing the effect of some of the polypeptide derivatives on weight change of rats;
FIG. 4 is a trend chart showing the effect of some of the polypeptide derivatives on accumulated food intake of rats;
FIG. 5 shows the plasma level of polypeptide derivatives D1 and N2; and
FIG. 6 shows the plasma level of some of the polypeptide derivatives as oral tablets.

## DETAILED DESCRIPTION

Illustrative examples of the present application are described as below, in which various details of the examples in the present application are included for ease of understanding and should be construed as illustrative only. Therefore, it is to be realized by those of ordinary skill in the art that many alterations and modifications may be made to the examples described here without departing from the scope and spirit of the present application. The following description also omits depiction of well known functions and structures for clarity and conciseness, and terms about technology and science used in this specification have the same meaning as generally understood by those skilled in the art, unless otherwise defined.

The terms "polypeptide", "peptide", and "protein" are used interchangeably herein and refer to an amino acid polymer of any length. The polymer can be linear or branched and may comprise naturally occurring amino acids among which non-naturally occurring ones may be interspersed.

The term "N terminus" refers to the N or amino terminal of a linear polypeptide consisting of a plurality of amino acids linked via peptide bonds and containing a free $\alpha$ amino group ($-NH_2$) and a free $\alpha$ carboxyl group (-COOH) that, when unmodified, are unbonded in respective amino acids at both ends, i.e., the terminus harboring an unbonded, free $\alpha$-$NH_2$ in the polypeptide. A modification site at the N terminus or an N terminal modification refers to making a modification to an amino acid at the N terminus of the polypeptide.

The term "derivative" herein refers to a product resulting from a modification of a functional group (e.g., an amino

acid residue) in a biomacromolecule (e.g., a polypeptide or protein) with a certain compound or molecule. The modification includes but is not limited to acylation, amidation, esterification, and thioesterification.

The term "activity selectivity" of a polypeptide or a polypeptide derivative herein refers to an attribute of the polypeptide or polypeptide derivative where it has a stronger effect on one of the receptors it acts on than another. In some embodiments of the present application, the activity refers to agonistic activity.

In some embodiments of the present application, the activity selectivity may further refer to preferentially improved activity of the polypeptide derivative against an amylin receptor over a calcitonin receptor. Preferably, the activity selectivity refers to an improved ratio of activity of the polypeptide derivative against an amylin receptor to that against a calcitonin receptor.

A pharmaceutical composition herein may be used for the treatment of diseases or for cell culture assays *in vitro.* When used for the treatment of diseases, the term "pharmaceutical composition" generally refers to a unit dosage form and may be prepared by any of the methods well known in the field of pharmaceutics. The methods used include a step of blending the active ingredients with excipients that are one or more auxiliary ingredients. Typically, a composition is prepared by uniformly and adequately blending active compounds with a liquid excipient, a fine solid excipient or both.

Being "pharmaceutically acceptable" herein refers to an attribute of a substance or composition that has to be chemically and/or toxicologically compatible with other ingredients contained in the formulation and/or mammals treated thereby.

The term "pharmaceutically acceptable excipient" herein may include any solvent, solid excipient, diluent or other liquid excipients or the like suitable for particular target dosage forms. Use of any conventional excipients is also within the scope contemplated in the present application, except to the extent that they are incompatible with the compounds of the present application, for example, result in any undesirable biological effects or interact with any other components of the pharmaceutically acceptable composition in a deleterious way.

The term "treatment" herein refers to the attainment of a desired pharmacological and/or physiological effect. The effect can be prophylactic in terms of completely or partially preventing a disease or a symptom thereof, and/or can be therapeutic in terms of completely or partially curing a disease and/or an adverse effect attributable to the disease. The "treatment" used herein encompasses, with respect to a disease in a mammal, particularly a human, (a) preventing occurrence of a disease or condition in an individual susceptible to but not diagnosed with the disease; (b) inhibiting a disease, for example, impeding the development of the disease; or (c) alleviating a disease, for example, relieving symptoms related to the disease. The "treatment" used herein encompasses any administration of a medicament or compound to an individual to treat, cure, alleviate, ameliorate, palliate or inhibit a disease of the individual, including but not limited to giving a medicament comprising the compound described herein to an individual in need thereof.

The term "prevention" herein refers to lowering the possibility of occurrence (or recurrence) of a disease, condition, or disorder or related symptoms (e.g., cancer).

## Abbreviations

Corresponding names or structures of some abbreviations used in the examples of the present application are as follows:

Trx:

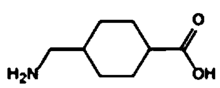

Inp:

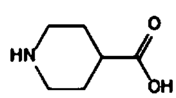

AEEA:

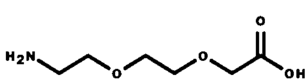

AA: Amino Acid; Boc: t-Butyloxy carbonyl; DCM: dichloromethane; DMF: N,N-Dimethyl formamide; DIEA: N,N-Diisopropylethylamine; EDT: 1,2-Ethanedithiol; Fmoc: 9-fluorenylmethyloxycarbonyl; OtBu: t-butyl ester; Pbf: 2,2,4,6,7-Pentamethyldihydrobenzofuran-5-sulfonyl chloride; Pip: Piperidine; TBTU: O-(Benzotriazol-1-yl)-N,N,N',N',-tetramethyluronium Tetrafluoroborate; tBu: tertiary butyl; TFA: Trifluoroacetic acid; TIS: Triisopropylsilane; Trt: Triphenylmethyl; αGlu: α-glutamic acid; yGlu: γ-glutamic acid; αAsp: α-aspartic acid; and βAsp: β-aspartic acid.

## EXAMPLES

### Example 1 Preparation of Polypeptide Derivatives

Polypeptides derivatives prepared in the present application are shown in Table 2.

Target products in Table 2 were prepared by solid phase organic synthesis, specifically, solid-phase peptide synthesis (SPPS) with Fmoc-protected amino acids, followed by cleavage, oxidization, and purification.

Taking the compound D1 (Cagrilintide) as an example, which has the structure of:

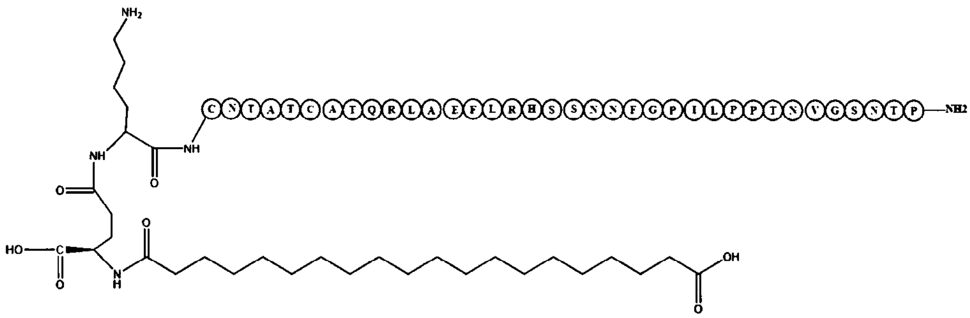

,

the synthesis process was as below:

1.1 Solid-phase synthesis

Using Fmoc-Linker MBHA Resin S=0.32 mmol/g and employing Fmoc/tBu processing, amino acids were linked by sequential condensation from the C terminus to the N terminus (from right to left) as per the sequence of the aforesaid peptide in accordance with the method provided in Table 1.

Table 1. Listing of synthesis procedures

| No. | Solvent | Number of times | duration (min/time) |
|---|---|---|---|
| 1 | 20% Pip/DMF | 1 | 10 |
| 2 | 20% Pip/DMF | 1 | 5 |
| 3 | DMF | 5 | 1 |
| 4 | Sampling for colorimetric testing | | |
| 5 | AA/DIEA/TBTU | 1 | 60 |
| 6 | DMF | 4 | 1 |
| 7 | Sampling for colorimetric testing | | |
| Notes | Repeating 1-7 till the end of the linear peptide synthesis | | |

The following amino acids were coupled in order:
A-01 Fmoc-Pro-OH, A-02 Fmoc-Thr(tBu)-OH, A-03 Fmoc-Asn(Trt)-OH, A-04 Fmoc-Ser(tBu)-OH, A-05 Fmoc-Gly-OH, A-06 Fmoc-Val-OH, A-07 Fmoc-Asn(Trt)-OH, A-08 Fmoc-Thr(tBu)-OH, A-09 Fmoc-Pro-OH, A-10 Fmoc-Pro-OH, A-11 Fmoc-Leu-OH, A-12 Fmoc-Ile-OH, A-13 Fmoc-Pro-OH, A-14 Fmoc-Gly-OH, A-15 Fmoc-Phe-OH, A-16 Fmoc-Asn(Trt)-OH, A-17 Fmoc-Asn(Trt)-OH, A-18 Fmoc-Ser(tBu)-OH, A-19 Fmoc-Ser(tBu)-OH, A-20 Fmoc-His(Trt)-OH, A-21 Fmoc-Arg(Pbf)-OH, A-22 Fmoc-Leu-OH, A-23 Fmoc-Phe-OH, A-24 Fmoc-Glu(OtBu)-OH, A-25 Fmoc-Ala-OH, A-26 Fmoc-Leu-OH, A-27 Fmoc-Arg(Pbf)-OH, A-28 Fmoc-Gln(Trt)-OH, A-29 Fmoc-Thr(tBu)-OH, A-30 Fmoc-Ala-OH, A-31 Fmoc-Cys(Trt)-OH, A-32 Fmoc-Thr(tBu)-OH, A-33 Fmoc-Ala-OH, A-34 Fmoc-Thr(tBu)-OH, A-35 Fmoc-Asn(Trt)-OH, A-36 Fmoc-Cys(Trt)-OH, A-37 Fmoc-Lys(Boc)-OH, A-38 Fmoc-Glu-otbu, and A-39 C20 diacid.

Finally, a polypeptide derivative-on-resin was formed.

The polypeptide derivative-on-resin was washed, transferred out, and dried to a constant weight for cleavage.

1.2 Cleavage

The cleavage reagent was used in an amount of 10 mL±2 mL per 1 g of the polypeptide derivative-on-resin. For cleavage, $H_2O$, TFA, EDT, and TIS composing the required cleavage reagent were sequentially added to a cleavage reaction flask in a ratio of TFA:$H_2O$:EDT:TIS = 95:1:2:2 and were controlled at a temperature of 0-10°C. The cleavage reagent was added to the resin under stirring, and after the temperature of the system became stable, the reactants were stirred for another 2.5 hours under a controlled temperature of 25-30°C. The cleavage solution was filtered, and allowed for precipitation using glacial diethyl ether of a 5x volume of the liquid. The precipitates were filtered, washed 3 times with glacial diethyl ether of a 3x volume of the liquid, and then dried under reduced pressure at room temperature to yield a solid crude.

1.3 Oxidation

The crude was finely ground, and was slowly added, with stirring, to purified water, while an aqueous solution of acetonitrile was added dropwise. After the crude was added and completely dissolved, a methanol solution of iodine was added and stirred for half an hour.

1.4 Purification and lyophilization

The oxidated liquid as described above was filtered with a 0.45 μm microporous filtering membrane. The crude was separated and purified using a column prepared by C-18 column packing material with a suitable gradient at ambient temperature, and then the target product was collected, detected and analyzed, and sorted. A purity ≥90% was required. Substandard target products were collected, and separated and purified again with a suitable gradient, to achieve qualified liquid peaks. The qualified liquid samples as mentioned above were lyophilized under reduced pressure to yield lyophilized powder of the refined polypeptide derivative.

Polypeptide derivatives prepared using a similar method to that described in Example 1 of the present application are shown in Table 2 and are distinguished from each other by the amino acid sequence and/or the fatty acid-containing side chain used in the solid-phase synthesis.

Table 2. Amino acid sequences and modifications of polypeptides and derivatives thereof

| ID | SEQ ID NO. | Amino acid sequence | Site of modification | Fatty acid |
|---|---|---|---|---|
| D1 | SEQ ID NO. 1 | KCNTATCATQ RLAEFLRHSS NNFGPILPPT NVGSNTP-$NH_2$ | N terminus | C20 diacid + γGlu |
| D2 | SEQ ID NO. 5 | CSNLS TCVLG KLSQE LHKLQ TYPRT NTGSG TP-$NH_2$ | None | None |
| N1 | SEQ ID NO. 2 | KCNTATCATQ RLADFLRHSS NNLKPILPPT NVGSNT-trans-Hyp-$NH_2$ | N terminus | C20 diacid +2*AEEAs |
| N2 | SEQ ID NO. 2 | KCNTATCATQ RLADFLRHSS NNLKPILPPT NVGSNT-trans-Hyp-$NH_2$ | N terminus | C20 diacid +βAsp +2*AEEAs |
| N3 | SEQ ID NO. 2 | KCNTATCATQ RLADFLRHSS NNLKPILPPT NVGSNT-trans-Hyp-$NH_2$ | N terminus | C18 diacid +βAsp +2*AEEAs |
| M1 | SEQ ID NO. 1 | KCNTATCATQ RLAEFLRHSS NNFGPILPPT NVGSNTP-$NH_2$ | N terminus | C18 diacid +yGlu |

(continued)

| ID | SEQ ID NO. | Amino acid sequence | Site of modification | Fatty acid |
|---|---|---|---|---|
| M2 | SEQ ID NO. 1 | KCNTATCATQ RLAEFLRHSS NNFGPILPPT NVGSNTP-$NH_2$ | N terminus | C20 diacid +2*AEEAs+yGlu |
| M3 | SEQ ID NO. 1 | KCNTATCATQ RLAEFLRHSS NNFGPILPPT NVGSNTP-$NH_2$ | N terminus | C18 diacid +2*AEEAs+γGlu |
| M5 | SEQ ID NO. 3 | ASQLS TAVLG RLSQE LHRLQ DYPRT DVGSG SP-$NH_2$ | None | None |
| M6 | SEQ ID NO. 4 | ASQLS TAVLG RLSDE LHRLQ DYPRT DVGSG SP-$NH_2$ | N terminus | C20 diacid + yGlu |
| M7 | SEQ ID NO. 4 | ASQLS TAVLG RLSDE LHRLQ DYPRT DVGSG SP-$NH_2$ | N terminus | C22 diacid + γGlu |
| M8 | SEQ ID NO. 4 | ASQLS TAVLG RLSDE LHRLQ DYPRT DVGSG SP-$NH_2$ | N terminus | C18 diacid + γGlu |
| M9 | SEQ ID NO. 4 | ASQLS TAVLG RLSDE LHRLQ DYPRT DVGSG SP-$NH_2$ | N terminus | C16 diacid + γGlu |
| M10 | SEQ ID NO. 4 | ASQLS TAVLG RLSDE LHRLQ DYPRT DVGSG SP-$NH_2$ | N terminus | C20 diacid + αGlu |
| M11 | SEQ ID NO. 4 | ASQLS TAVLG RLSDE LHRLQ DYPRT DVGSG SP-$NH_2$ | N terminus | C18 diacid + αGlu |
| M12 | SEQ ID NO. 4 | ASQLS TAVLG RLSDE LHRLQ DYPRT DVGSG SP-$NH_2$ | N terminus | C20 diacid + βAsp |
| M13 | SEQ ID NO. 4 | ASQLS TAVLG RLSDE LHRLQ DYPRT DVGSG SP-$NH_2$ | N terminus | C18 diacid + αAsp |
| M14 | SEQ ID NO. 4 | ASQLS TAVLG RLSDE LHRLQ DYPRT DVGSG SP-$NH_2$ | N terminus | C20 diacid + Trx |
| M15 | SEQ ID NO. 4 | ASQLS TAVLG RLSDE LHRLQ DYPRT DVGSG SP-$NH_2$ | N terminus | C18 diacid + Trx |
| M16 | SEQ ID NO. 4 | ASQLS TAVLG RLSDE LHRLQ DYPRT DVGSG SP-$NH_2$ | N terminus | C20 diacid + Inp |
| M17 | SEQ ID NO. 4 | ASQLS TAVLG RLSDE LHRLQ DYPRT DVGSG SP-$NH_2$ | N terminus | C18 diacid + Inp |
| M18 | SEQ ID NO. 4 | ASQLS TAVLG RLSDE LHRLQ DYPRT DVGSG SP-$NH_2$ | N terminus | C20 diacid + yGlu+2 AEEAs |
| M19 | SEQ ID NO. 4 | ASQLS TAVLG RLSDE LHRLQ DYPRT DVGSG SP-$NH_2$ | N terminus | C20 diacid + yGlu+4 AEEAs |

(continued)

| ID | SEQ ID NO. | Amino acid sequence | Site of modification | Fatty acid |
|---|---|---|---|---|
| M20 | SEQ ID NO. 4 | ASQLS TAVLG RLSDE LHRLQ DYPRT DVGSG SP-$NH_2$ | N terminus | C20 diacid + γGlu+6 AEEAs |
| M21 | SEQ ID NO. 4 | ASQLS TAVLG RLSDE LHRLQ DYPRT DVGSG SP-$NH_2$ | N terminus | C20 diacid + yGlu+8 AEEAs |
| M22 | SEQ ID NO. 4 | ASQLS TAVLG RLSDE LHRLQ DYPRT DVGSG SP-$NH_2$ | N terminus | C18 **diacid** + yGlu+2 AEEAs |
| M23 | SEQ ID NO. 4 | ASQLS TAVLG RLSDE LHRLQ DYPRT DVGSG SP-$NH_2$ | N terminus | C20 diacid + αGlu+2 AEEAs |
| M24 | SEQ ID NO. 4 | ASQLS TAVLG RLSDE LHRLQ DYPRT DVGSG SP-$NH_2$ | N terminus | C18 diacid + αGlu+2 AEEAs |
| M25 | SEQ ID NO. 4 | ASQLS TAVLG RLSDE LHRLQ DYPRT DVGSG SP-$NH_2$ | N terminus | C20 diacid + βAsp+2 AEEAs |
| M26 | SEQ ID NO. 4 | ASQLS TAVLG RLSDE LHRLQ DYPRT DVGSG SP-$NH_2$ | N terminus | C18 diacid + αAsp+2 AEEAs |
| M27 | SEQ ID NO. 4 | ASQLS TAVLG RLSDE LHRLQ DYPRT DVGSG SP-$NH_2$ | N terminus | C20 diacid + Trx+2 AEEAs |
| M28 | SEQ ID NO. 4 | ASQLS TAVLG RLSDE LHRLQ DYPRT DVGSG SP-$NH_2$ | N terminus | **C18 diacid** + Trx+2 AEEAs |
| M29 | SEQ ID NO. 4 | ASQLS TAVLG RLSDE LHRLQ DYPRT DVGSG SP-$NH_2$ | N terminus | C20 diacid + Inp+2 AEEAs |
| M30 | SEQ ID NO. 4 | ASQLS TAVLG RLSDE LHRLQ DYPRT DVGSG SP-$NH_2$ | N terminus | C18 diacid + Inp+2 AEEAs |

N1-N3 or M1-M30 represent derivatives resulting from modifications of the amino acid sequences set forth in SEQ ID No. 1-SEQ ID No. 5 with the corresponding fatty acid-containing side chains in Table 2, or are polypeptides otherwise (where there is no fatty acid modification). The fatty acid-containing side chains are in the form of Z1+Z2+Z3, wherein Z1 is a C16-C22 fatty diacid; Z2 is selected from any one of γGlu, αGlu, βAsp, αAsp, Inp, and Trx, or is absent; Z3 is 0-8, for example 0, 1, 2, 3, 4, 5, or 6, AEEA(s); and Z1, Z2, and Z3 are linked via amide linkages. The C16-C22 fatty diacid moiety is located at the outer side of the fatty acid-containing side chain for modification relative to the amino acid sequence, and thus is positioned at the distal end of the linkage of the side chain to the amino acid sequence. For instance, M1 represents a derivative offered by modifying the amino acid sequence set forth in SEQ ID No. 1 with the fatty acid-containing side chain "C18 diacid+γGlu", wherein the γGlu end of the side chain is linked to the amino acid sequence via a peptide bond and the C18 fatty diacid end is disposed at the distal end of the linkage of the side chain to the amino acid sequence.

**Example 2 Testing for Activity of the Polypeptide Derivatives *in vitro***

The objective of this assay is to test the potency of the polypeptide derivatives in the present application on amylin and calcitonin receptors *in vitro* using a luciferase assay.

2.1 Amylin receptor agonism testing

Using the standard protocol for construction of an amylin receptor/CRE-luc cell line, CHO-K1/Ga15/AMY3 cells (purchased from GenScript, with the calcitonin receptor and the receptor activity modifying peptide (RAMP) constructed already) were transfected with a plasmid containing a luciferase expression cassette driven by multiple copies of cAMP response elements (CREs). The cells were cultured in an F12 medium containing 200 ug/mL Zeocin, 2 ug/mL puromycin, 100 ug/mL hygromycin, and 400 ug/mL G418 to afford a stably transfected amylin receptor/CRE-luc cell line.

For the luciferase assay of amylin, the lyophilized powder obtained according to the method of Example 1 was dissolved in 20 mM phosphate buffer solution with a pH of 7.0, and diluted in a growth medium (F12 culture medium containing 10% FBS) to give a derivative sample with an initial concentration of 10 nM, which was then subjected to a 5-fold serial dilution in the growth medium to afford samples of 7 concentrations including 10 nM, 2 nM, 0.4 nM, etc. To each well of a white 96-well plate was added 50 μL of the tested sample solution at a respective concentration.

The stably transfected amylin receptor/CRE-luc CHO cells were resuspended in the growth medium at a certain density, and 50 μL of the cell suspension was added at a density of about 20,000 cells per well to the white 96-well plate containing the tested sample solution. After incubation of 24 hours at 37°C and 5% $CO_2$, 100 μL of a Luciferase substrate was added to each well, incubated for 3 minutes, and finally was tested for luminescence on SpectraMax L (Molecular Devices) with the software SoftMax Pro 7.0.3 GxP. A standard curve was plotted using the fluorescent intensities for calculation of EC50.

Samples of compounds N2 and N3 were tested following the same method as the above, except that the tested sample solutions and the CHO cells were subjected to a condition where "after incubation of 4 hours at 37°C and 5% $CO_2$, 100 μL of a Luciferase substrate was added to each well".

Results were shown in the Table 3.

Table 3. Results for activity against the amylin receptor in cells

| ID | EC50/nM | ID | EC50/nM |
|---|---|---|---|
| D1 | 0.036 | M21 | 0.044 |
| M5 | 0.033 | M22 | 0.015 |
| M6 | 0.017 | M23 | 0.017 |
| M7 | 0.022 | M24 | 0.007 |
| M8 | 0.016 | M25 | 0.017 |
| M9 | 0.002 | M26 | 0.008 |
| M10 | 0.069 | M27 | 0.004 |
| M11 | 0.016 | M28 | 0.002 |
| M12 | 0.017 | M29 | 0.006 |
| M13 | 0.016 | M30 | 0.001 |
| M14 | 0.456 | N1 | 0.016 |
| M15 | 0.119 | N2 | 0.04 |
| M16 | 0.082 | / | / |
| M17 | 0.063 | / | / |
| N2 | 0.009 | N3 | 0.001 |

Data in Table 3 showed that the polypeptide derivatives of the present application had high activity in cells and exhibited great agonistic activity against the amylin receptor.

2.2 Calcitonin receptor agonism

2.2.1 Construction of a calcitonin receptor/CRE-luc cell line

Using the standard protocol, CHO-K1/CALCR/Gα15 cells (purchased from GenScript, with the calcitonin receptor constructed already) were transfected with a plasmid containing a luciferase expression cassette driven by multiple copies of cAMP response elements (CREs). The cells were cultured in an F12 medium containing 200 ug/mL

Zeocin, 2 ug/mL puromycin, and 100 ug/mL hygromycin to afford a stably transfected calcitonin receptor/CRE-luc cell line.

2.2.2 Luciferase assay of calcitonin

The lyophilized powder from Example 1 was dissolved in 20 mM phosphate buffer solution with a pH of 7.0, and diluted in a growth medium (F12 culture medium containing 10% FBS) to give a derivative sample with an initial concentration of 10 nM, which was then subjected to a 5-fold serial dilution in the growth medium to afford samples of 7 concentrations including 10 nM, 2 nM, 0.4 nM, etc. To each well of a white 96-well plate was added 50 μL of the tested sample solution at a respective concentration.

The stably transfected calcitonin receptor/CRE-luc CHO cells were resuspended in the growth medium at a certain density, and 50 μL of the cell suspension was added at a density of about 20,000 cells per well to the white 96-well plate containing the tested sample solution. After incubation of 24 hours at 37°C and 5% $CO_2$, 100 μL of a Luciferase substrate was added to each well, incubated for 3 minutes, and finally was tested for luminescence on SpectraMax L (Molecular Devices) with the software SoftMax Pro 7.0.3 GxP. A standard curve was plotted using the fluorescent intensities for calculation of EC50. Results were shown in the following table.

Table 4. Results for activity against the calcitonin receptor in cells

| ID | EC50/nM | ID | EC50/nM | ID | EC50/nM |
|---|---|---|---|---|---|
| D1 | 0.086 | M12 | 0.073 | M18 | 0.069 |
| M6 | 0.028 | M13 | 0.085 | M25 | 0.138 |
| M7 | 0.194 | M14 | 0.587 | M24 | 0.081 |
| M8 | 0.165 | M15 | 0.469 | M26 | 0.024 |
| M9 | 0.120 | M16 | 0.376 | | |
| M11 | 0.071 | M17 | 0.259 | | |

Data in Table 4 showed that the polypeptide derivatives of the present application had high activity in cells and exhibited agonistic activity against the calcitonin receptor.

An integrated analysis of data of some polypeptide derivatives of the present application on activity against the amylin receptor (hAMY3R) and the calcitonin receptor (hCTR) in cells was shown in Table 5. Compared with a polypeptide derivative with a fatty acid-containing side chain in the form of Z1+Z2 (i.e., fatty diacid + amino acid), providing activity of the molecule D1 against hAMY3R and hCTR in cells as a benchmark, the polypeptide derivatives of the present application comprising a fatty acid-containing side chain in the form of Z1+Z2+Z3 (i.e., fatty diacid + amino acid + AEEA) showed a surprisingly significant difference in activity against the amylin and calcitonin receptors in cells and exhibited greater agonistic activity/relative activity against hAMY3R than hCTR, and such improvement in activity was especially pronounced when Z2 was Trx or Inp. For instance, M30 (SEQ ID NO. 4 modified with C18 diacid + Inp +2 AEEAs) had agonistic activity against the amylin receptor increased by nearly 70 folds as compared with M17 (SEQ ID NO. 4 modified with C18 diacid + Inp) and showed an about 42.5-fold increase in the ratio of activity against the amylin receptor to agonistic activity against the calcitonin receptor as opposed to the 1.7-fold increase achieved by M17 relative to the benchmark, displaying a stronger tendency for amylin receptor agonism.

Similarly, another derivative N3 comprising a different amino acid sequence (SEQ ID NO. 2) modified by the fatty acid-containing side chain in the form of Z1+Z2+Z3 (i.e., fatty diacid + amino acid + AEEA) also exhibited high agonistic activity against the amylin receptor.

Table 5. Data on activity against the amylin receptor and the calcitonin receptor in cells

| ID | Fatty acid | hAMY3R EC50/nM | hCTR EC50/nM | Fold change in activity against hAMY3R | Fold change in activity against hCTR |
|---|---|---|---|---|---|
| D1 | C20 diacid + γGlu | 0.036 | 0.086 | 1.000 | 1.000 |
| M6 | C20 diacid + γGlu | 0.017 | 0.028 | 2.118 | 3.071 |
| M18 | C20 diacid + γGlu+2 AEEAs | 0.016 | 0.069 | 2.25 | 1.246 |
| M12 | C20 diacid + βAsp | 0.017 | 0.073 | 2.118 | 1.178 |
| M25 | C20 diacid + βAsp+2 AEEAs | 0.017 | 0.138 | 2.118 | 0.623 |

(continued)

| ID | Fatty acid | hAMY3R EC50/nM | hCTR EC50/nM | Fold change in activity against hAMY3R | Fold change in activity against hCTR |
|---|---|---|---|---|---|
| M14 | C20 diacid + Trx | 0.456 | 0.587 | 0.079 | 0.147 |
| M27 | C20 diacid + Trx+2 AEEAs | 0.004 | 0.533 | 9.000 | 0.161 |
| M16 | C20 diacid + Inp | 0.082 | 0.376 | 0.439 | 0.229 |
| M29 | C20 diacid + Inp+2 AEEAs | 0.006 | 0.156 | 6.000 | 0.551 |
| M11 | C18 diacid + αGlu | 0.016 | 0.071 | 2.250 | 1.211 |
| M24 | C18 diacid + αGlu+2 AEEAs | 0.007 | 0.081 | 5.143 | 1.061 |
| M15 | C18 diacid + Trx | 0.119 | 0.469 | 0.303 | 0.183 |
| M28 | C18 diacid + Trx+2 AEEAs | 0.002 | 0.089 | 18.000 | 0.966 |
| M17 | C18 diacid + Inp | 0.063 | 0.259 | 0.571 | 0.332 |
| M30 | C18 diacid + Inp+2 AEEAs | 0.001 | 0.101 | 36.000 | 0.848 |

**Example 3 Stability Study of the Polypeptide Derivatives**

3.1 Materials and devices

A stability test chamber (BINDER GmbH), a 0.01 mg-readability balance (METTLER TOLEDO), a pH meter (METTLER TOLEDO), a biosafety cabinet (ESCO), T2G-II small-sized motor-driven capping machine (Changsha zhongya pharmaceutical equipment co. LTD), a highspeed refrigerated centrifuge (Eppendorf), a sterilizer cabinet (Shinva Medical), Agilent 1260 high-performance liquid chromatographer, and Sepax Bio-C18 4.6*250 mm 3 μm 200 Å reversed phase column were used.

Reagents and consumables included: ultrapure water (18.2 MΩ, self-made), acetonitrile (HPLC grade), trifluoroacetic acid (HPLC grade), sodium dihydrogen phosphate (pharmaceutical grade, Hunan Jiudian Hongyang Pharmaceutical Co., Ltd), hydrochloric acid (pharmaceutical grade, Hunan Er-Kang Pharmaceutical Co., Ltd), sodium hydroxide (Analytical Reagent grade, Hushi®, Sinopharm Chemical Reagent Co., Ltd), vials, rubber stoppers, disposable syringes, and sterile filters.

3.2 Methods of the assay

The lyophilized powder (1 mg/mL) of the polypeptide derivative prepared in accordance with the method of Example 1 was mixed with sodium dihydrogen phosphate (1.42 mg/mL) and dissolved in ultrapure water in the indicated mass-volume ratios. After adjusting pH to about 7.4 with hydrochloric acid/sodium hydroxide, the solution was filtered into a sterilized vial in a clean bench using a 0.22 μm sterile filter. The vial was then capped and placed into the stability test chamber at 40°C. Detections were made on Days 7 (7d) and 26 (26d), and during the assay, changes in the characteristics and properties of the polypeptide were observed and recorded. The sample was then centrifuged for 3 min at 10,000 rpm at 4°C, and the supernatant was transferred to a liquid phase sample vial for detecting the concentration and purity of the polypeptides using liquid phase chromatography described as below, wherein the concentration of the polypeptide = peak area/injection volume/extinction coefficient/60×flow rate, and the purity of the polypeptide = target peak area/total peak area×100%.

Conditions of the reversed phase chromatography Flow rate: 1.0 ml/min; Autosampler temperature: 15°C; Column temperature: 25°C; Detection wavelengths: 280 nm and 214 nm; Mobile phase A: 100% $H_2O$+0.05% TFA; Mobile phase B: 100% ACN; and Gradient of elution listed in Table 6:

Table 6. Listing of the gradient of elution in the liquid phase chromatography

| Time (min) | 0 | 3 | 8 | 13 | 14 | 20 |
|---|---|---|---|---|---|---|
| B% | 25 | 25 | 70 | 70 | 25 | 25 |

3.3 Results of the assay

As was observed in the heat accelerated stability assay, the solutions of the polypeptide derivatives of the present application remained to be clear on Day 7 of the heat accelerated assay, and exhibited distinctly higher clarity than the solution of the molecule D1 in which foreign matters were readily observable on Day 26 of the assay.

Table 7 demonstrated that there was no obvious change in concentration of the polypeptide derivatives provided in the present application during the heat accelerated stability assay under a neutral condition. In contrast to the molecule D2 showing a change of -40% in concentration on Day 7 of the accelerated testing, the present polypeptide derivatives underwent minimal changes in concentration, some of which (e.g., M6, M19, M22, M23, M24, M27, M29, and M30) even had a concentration change of less than 1% (denoted as "no change"). Concentration changes of solutions of the polypeptide derivatives of the present application remained below 10% even on Day 26 of the heat accelerated assay, and some were yet lower, standing at less than 3%.

Table 7. Concentration changes in the heat accelerated stability assay

| ID | Concentration change on 7d/% | Concentration change on 26d | ID | Concentration change on 7d | Concentration change on 26d |
|---|---|---|---|---|---|
| D2 | -40.064 | / | M18 | No change | -1.148 |
| M6 | No change | -1.865 | M19 | No change | -1.589 |
| M7 | No change | No change | M20 | No change | -3.662 |
| M8 | No change | -6.190 | M21 | -1.388 | -4.522 |
| M9 | -2.280 | -7.796 | M22 | 1.314 | -5.493 |
| M10 | -2.290 | -10.562 | M23 | -4.443 | -7.782 |
| M11 | -3.472 | -9.840 | M24 | No change | -3.954 |
| M12 | No change | -8.378 | M25 | -1.222 | -2.645 |
| M13 | -1.895 | -5.520 | M26 | -2.402 | -2.235 |
| M14 | -3.464 | -8.377 | M27 | No change | -2.286 |
| M15 | No change | -10.824 | M29 | No change | No change |
| M16 | -2.883 | -3.036 | M30 | -1.805 | -5.268 |
| M17 | -1.927 | -5.985 | | | |
| Note: concentration change = (concentration on 7d or 26d - concentration on 0d)/concentration on 0d×100% | | | | | |

Table 8 revealed that there was no significant change in purity of the polypeptide derivatives provided in the present application during the heat accelerated stability assay under a neutral condition, as determined by the reversed phase chromatography. On Day 7 of the accelerated testing, purity changes of most of the polypeptide solutions were less than 1% when determined as above, or even no purity decrease was observed, and the polypeptide derivatives of the present application maintained a high purity whereas the molecule D1 in the prior art showed a purity decrease of 12.73% on Day 7 of the accelerated testing. Molecules such as M7, M18, M19, and M23 were further found to have nearly no noticeable change in purity even on Day 26 of the accelerated testing, showing great stability in purity.

Table 8. Purity changes in the heat accelerated stability assay (Purity from the reversed phase chromatography-214 nm)

| ID | Purity change on 7d | Purity change on 26d | ID | Purity change on 7d | Purity change on 26d |
|---|---|---|---|---|---|
| D1 | -12.73 | / | M18 | No change | No change |
| M6 | 1.64 | No change | M19 | No change | No change |
| M7 | No change | No change | M20 | No change | -1.59 |
| M8 | No change | -3.3 | M21 | -1.66 | -2.01 |
| M9 | -1.67 | -4.82 | M22 | 2.59 | -2.19 |
| M10 | -1.66 | -5.98 | M23 | No change | No change |

(continued)

| ID | Purity change on 7d | Purity change on 26d | ID | Purity change on 7d | Purity change on 26d |
|---|---|---|---|---|---|
| M11 | 1.01 | -2.93 | M24 | -1.59 | -7.24 |
| M12 | 1.12 | -1.96 | M25 | -2.45 | -5.24 |
| M13 | -1.15 | -3.76 | M26 | -2.39 | -2.68 |
| M14 | No change | -1.77 | M27 | No change | -1.27 |
| M15 | No change | -5.67 | M29 | No change | -1.2 |
| M16 | 3.42 | 2.18 | M30 | No change | -4.34 |
| M17 | -1 | -3.9 | | | |
| Note: $\text{purity change} = \text{purity on 7d or 26d} - \text{purity on 0d}$ | | | | | |

**Example 4 Results of Animal Study**

SD (Sprague Dawley) rats weighing 200-250 g were used for this study. The rats were received at least 10-14 days before the study began such that they could accommodate to the experimental environment. After arrival, they were exposed to a reversed light/dark cycle (i.e., lights switched off in daytime and on in nighttime) for two weeks and were individually housed for the first week so as to ensure high data accuracy and test sensitivity. Throughout the acclimation and experiment period, the rats had free access to food and water. 5-8 rats were assigned to each testing group for the derivatives and were subcutaneously administered the derivatives at a dose of 10 nmol/kg or a vehicle (20 mM phosphate buffer, PB, pH 7.0), and the dosing timepoint was recorded for each group. After dosing, the rats were put back into cages where they were housed and could have access to food and water. Food consumption and weight change of the rats were recorded online or manually every 24 hours. Percent of weight change= $(BWT_n-BWT_0)/BWT_0*100\%$, wherein BW represented the value of body weight, and $T_0$ and $T_n$ represented the time point of dosing and n hours post dose respectively. Dots in the figures were represented utilizing mean ± standard error of the mean (SEM).

As shown in FIGs. 1-4, the polypeptide derivatives of the present application exerted an obviously superior effect on reducing both body weight and accumulated food intake in the SD rats as compared to the control (the vehicle or D1). More importantly, the addition of AEEA to the linker, e.g., in the polypeptide derivatives M18, M23, M25, M27, and M29, enabled apparently further reduced body weight and accumulated food intake in the SD rats as compared with a single amino acid linker in molecules with the same polypeptide structure.

**Example 5 Efficacy Assay in SD Rats-A Dose-dependent Study**

SPF male SD rats (7-8 weeks, 220-230 g) were employed in this study after health inspection and quarantine. The experimental animals were exposed to a room temperature of 20°C-23°C and a relative humidity of 40%-50%, and were provided with Co60 mouse reproduction feed 1035 and purified water supplied with water feeding bottles for free intake throughout the health inspection and quarantine and the study.

In the assay for efficacy comparison between compounds D1 and N3, the animals were assigned to 5 groups of n=5 based on their mean body weight, namely, the vehicle group (20 mM phosphate buffer, PB, pH 7.0), the compound D1 groups of varying doses (30 nmol/kg and 100 nmol/kg), and the compound N3 groups of varying doses (30 nmol/kg and 100 nmol/kg).

In the assay for efficacy comparison between compounds N2 and N3, the animals were assigned to 7 groups of n=5 based on their mean body weight, namely, the vehicle group (20 mM phosphate buffer, PB, pH 7.0), the compound N2 groups of varying doses (5 nmol/kg, 25 nmol/kg, and 125 nmol/kg), and the compound N3 groups of varying doses (5 nmol/kg, 25 nmol/kg, and 125 nmol/kg).

The animals were administered a single dose via subcutaneous injection in the study, wherein the day of administration was designated as Day 1/Time 0 (T0), the initial body weight of the animals were recorded and the initial feed was added on Day 0, and the body weight of the studied animals as well as the remaining food were recorded daily for calculating the percentage of weight change and the amount of food intake, any anomalies during which were required to be recorded and reported. During the study, the rats were in good condition and no anomaly was observed. Percent of weight change= $(BWT_n-BWT_0)/BWT_0*100\%$, wherein BW represented the value of body weight, and $T_0$ and $T_n$ represented the time point of dosing and n hours post dose respectively. Dots in the figures were represented utilizing

mean ± standard error of the mean (SEM). Food intake was the amount of food consumed by each of the animals per day.

Table 9.

| Group | 24h weight change (%) | 24h food intake (g) |
|---|---|---|
| Vehicle | 2.095 | 25.000 |
| D1, 30 nmol/kg dose group | -6.416 | 5.250 |
| D1, 100 nmol/kg dose group | -9.103 | 2.100 |
| N3, 30 nmol/kg dose group | -8.422 | 2.100 |
| N3, 100 nmol/kg dose group | -11.276 | 0.500 |

Table 10.

| Group | 24h weight change (%) | 24h food intake (g) |
|---|---|---|
| Vehicle | 4.712 | 25.610 |
| N2, 5 nmol/kg dose group | 0.688 | 18.595 |
| N2, 25 nmol/kg dose group | -3.188 | 9.280 |
| N2,125 nmol/kg dose group | -7.110 | 1.585 |
| N3, 5 nmol/kg dose group | -0.316 | 11.920 |
| N3, 25 nmol/kg dose group | -3.822 | 6.735 |
| N3,125 nmol/kg dose group | -9.000 | 0.120 |

The compound N2 had a significantly greater effect on reducing body weight than D1 (see Example 3 in CN 202310614769.1). Table 9 showed that N3 also obviously exhibited an enhanced effect on reducing body weight and accumulated food intake in the SD rats in a dose-dependent manner as compared with D1. N2 and N3 are additional derivatives comprising a different amino acid sequence (SEQ ID NO. 2) modified by the fatty acid-containing side chain in the form of Z1+Z2+Z3 (i.e., fatty diacid + amino acid + AEEA), in the comparison of which N3 unexpectedly showed a stronger effect on reducing body weight and food intake of the animals (Table 10).

**Example 6 Study on PK profile of Amylin Derivative Tablets**

The amylin derivatives were formulated as oral tablets, which further comprise the same amount of the oral delivery agent PNAC with a structure of:

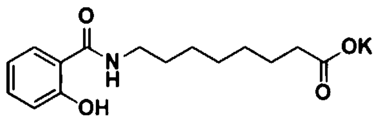

.

N-[8-(2-hydroxybenzoyl)amino]caprylic acid (NAC) was prepared utilizing the method described in Example 1 of the International Patent Application No. WO2008/028859. Isopropanol (22070.0 ml, 4.0 vol) was added to a 50 L reactor and stirred, and then NAC (5518 g, 1.0 eq) was added. After the system was warmed to 50°C, a 50% solution of potassium hydroxide (1304.0 g, 1.0 eq) was added dropwise, finally giving a clear yellow solution, which was allowed for reaction at 50°C for 1 h. The reaction solution was then concentrated at 40°C in portions to yield a light orange crude.

The combined crude was added to isopropanol (19310.0 ml, 3.5 vol), triturated for 1 h, and then subjected to suction filtration. The filter cake was rinsed with isopropanol (2760.0 ml, 0.5 vol), transferred to a vacuum dryer, pressure-balanced with nitrogen, dried for 16 h at 60°C, and then transferred to a vacuum dryer again and dried for 24 h at 100°C. After drying, a solid PNAC product of 4.52 kg in total was obtained as off-white powder with a yield of 72.8%.

Table 11 showed the amount of essential ingredients contained in tablets of the polypeptide derivatives comprising a fatty acid-containing side chain.

Table 11. An example of tablets of the polypeptide derivatives prepared in the present application

| Formulation | API (polypeptide) | Delivery agent (PNAC) | Lubricant (magnesium stearate for example) | Total mass |
|---|---|---|---|---|
| | Mass per tablet (mg) | Mass per tablet (mg) | Mass per tablet (mg) | Mass per tablet (mg) |
| 1 | 7 | 300 | 8 | 315 |

The tablets of the polypeptide derivatives comprising a fatty acid-containing side chain were prepared by sieving the polypeptide derivatives and PNAC, mixing them with excipients uniformly, and performing direct compression. Male Beagle dogs aged 10-15 months (9-12 kg) were each orally administered amylin derivatives (D1 and N2) at a dose of 7 mg once daily for 5 consecutive days (n=5), with the first day of oral administration of the protein molecule-based drugs designated as Day 1, and the last day as Day 5. Whole blood of the animals was collected pre dose (-10 min) and 2h, 4h, and 8h post dose on Day 1, pre dose (-10 min) and 2h and 4h post dose on Day 2-Day 4, and pre dose (-10 min) and 2h, 4h, 8h, 24h, 48h, and 72h post dose on Day 5, and processed into plasma using heparin sodium as an anticoagulant. The plasma was stored at -80°C for subsequent analysis of the plasma level of APIs using LC-MS/MS (Waters ACQUITY I Class Premier UPLC tandem with Sciex 6500+QQQ), wherein the charge-to-mass ratio of the ion pair for D1 was 1102.9/1074.5, and that for N2 was 945.6/919.8. Experimental data was plotted using GraphPad Prism9.3.1.

The results were shown in FIG. 5, demonstrating that compared with the tablet comprising the compound D1, the molecule of the compound N2 showed a special synergy with the delivery agent PNAC in the tablet, exhibiting an extraordinarily high oral bioavailability.

In the comparison of oral bioavailability between D1 and N3, N3 and the delivery agent PNAC in the tablet also produced the synergistic effect of significantly improving the bioavailability.

**Example 7 Study on Influence of Side Chain Modification on PK profile of Oral Tablets of the Polypeptide Derivatives**

Additional polypeptide analogs of amylin comprising a different sequence were used to further verify the influence of side chain modification on oral bioavailability of the polypeptide derivatives. Oral tablets of polypeptide derivatives D1, M2, and M3 shown in Table 1, which only differed by the side chain, were tested for their PK profile using a similar method to that described in Example 6.

The results were shown in FIG. 6, revealing that the polypeptide derivatives M2 and M3 enabled apparently higher absorption than the molecule D1 after oral administration, which was similar to the result in Example 6 and further indicated that modification with the side chain structures provided in the present application contributed effectively to improvement in oral bioavailability of the polypeptide derivatives, and M3 containing a C18 fatty acid had higher oral bioavailability than M2 containing a C20 fatty acid.

Although embodiments of the present application have been described as above, the present application is not limited to the above specific embodiments, which are only illustrative and directory but not restrictive, and application fields. Without departing from the scope of the claims of the present application, many variants may occur to those of ordinary skill in the art with inspiration from this specification.

## Claims

1. Use of a fatty acid-containing side chain in improving activity selectivity of a polypeptide derivative, wherein the polypeptide derivative is formed by modification of a polypeptide molecule comprising the fatty acid-containing side chain, wherein the fatty acid-containing side chain is in the form of Z1+Z2+Z3, wherein Z1 is a C16-C22 fatty diacid;

   Z2 is selected from any one of $\gamma$Glu, $\alpha$Glu, $\beta$Asp, $\alpha$Asp, Inp, and Trx, or is absent; and
   Z3 is n AEEA(s), wherein $n \geq 0$.

2. Use of a fatty acid-containing side chain in improving activity of a polypeptide derivative, wherein the polypeptide derivative is formed by modification of a polypeptide molecule comprising the fatty acid-containing side chain, wherein the fatty acid-containing side chain is in the form of Z1+Z2+Z3, wherein Z1 is a C16-C22 fatty diacid;

   Z2 is selected from any one of $\gamma$Glu, $\alpha$Glu, $\beta$Asp, $\alpha$Asp, Inp, and Trx, or is absent; and
   Z3 is n AEEA(s), wherein $n \geq 0$.

3. Use of a fatty acid-containing side chain in improving oral bioavailability of a polypeptide derivative, wherein the polypeptide derivative is formed by modification of a polypeptide molecule comprising the fatty acid-containing side chain, wherein the fatty acid-containing side chain is in the form of Z1+Z2+Z3, wherein Z1 is a C16-C22 fatty diacid;

   Z2 is selected from any one of γGlu, αGlu, βAsp, αAsp, Inp, and Trx, or is absent; and
   Z3 is n AEEA(s), wherein n≥0.

4. A method of improving activity selectivity of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

   Z2 is selected from any one of γGlu, αGlu, βAsp, αAsp, Inp, and Trx, or is absent; and
   Z3 is n AEEA(s), wherein n≥0.

5. A method of improving activity of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

   Z2 is selected from any one of γGlu, αGlu, βAsp, αAsp, Inp, and Trx, or is absent; and
   Z3 is n AEEA(s), wherein n≥0.

6. A method of improving oral bioavailability of a polypeptide derivative, comprising modifying a polypeptide molecule comprising a fatty acid-containing side chain to form the polypeptide derivative, wherein the fatty acid-containing side chain is in the form of Z1+Z2+Z3, wherein Z1 is a C16-C22 fatty diacid;

   Z2 is selected from any one of γGlu, αGlu, βAsp, αAsp, Inp, and Trx, or is absent; and
   Z3 is n AEEA(s), wherein n≥0.

7. The use of any one of claims 1-3 or the method of any one of claims 4-6, wherein Z1 is a C16, C17, C18, C19 or C20 fatty diacid.

8. The use or the method of any one of claims 1-7, wherein Z2 is selected from any one of γGlu, βAsp, γGlu, Inp, and Trx, preferably βAsp or γGlu.

9. The use or the method of any one of claims 1-8, wherein n is greater than 0, preferably 1-10, more preferably 2-8, and further preferably, n=2.

10. The use or the method of any one of claims 1-9, wherein Z1 is a C16, C18 or C20 fatty diacid.

11. The use or the method of any one of claims 1-10, wherein Z2 is Inp or Trx; and n is greater than 0.

12. The use or the method of any one of claims 1-11, wherein Z1 is a C16, C17, C18, C19 or C20 fatty diacid; Z2 is γGlu; and n=2.

13. The use or the method of any one of claims 1-12, wherein Z1, Z2, and Z3 are linked via amide linkages.

14. The use or the method of any one of claims 1-13, wherein the modification of the polypeptide molecule comprising the side chain is on 1-10 amino acids, preferably 1-3 amino acids, and more preferably 1 amino acid, of the polypeptide.

15. The use or the method of any one of claims 1-14, wherein the modification of the polypeptide molecule comprising the side chain is on an amino acid at the N terminus or any middle position of the polypeptide, wherein the amino acid is preferably selected from glutamic acid, aspartic acid, lysine, glutamine, or asparagine, and is further preferably lysine.

16. The use or the method of any one of claims 1-15, wherein the polypeptide is natural or artificially synthetic polypeptide molecule, wherein the polypeptide is an agonist or an antagonist, preferably wherein the polypeptide is selected from a GLP-1 receptor agonist, a GIP receptor antagonist, an insulin receptor agonist, a heparin receptor agonist, somatotrophin, an interferon receptor agonist, an interleukin receptor agonist, a follicle-stimulating hormone receptor agonist, a gonadotropin receptor agonist, an erythropoietin receptor agonist, a Peptide YY (PYY) receptor agonist, an

oxygenation modulator receptor agonist, a glucagon-like peptide-2 (GLP-2) receptor agonist, a calcitonin receptor agonist, a parathyroid hormone (PTH) receptor agonist, a ghrelin receptor agonist, an endocannabinoid receptor agonist, a leptin receptor agonist, a serotonin receptor agonist, a fibroblast growth factor 21 (FGF21) receptor agonist, a cholecystokinin (CCK) receptor agonist, an oxyntomodulin receptor agonist, or a glucagon receptor agonist, preferably an amylin receptor agonist, and further preferably an amylin or calcitonin analog.

**17.** The use or the method of any one of claims 1-16, wherein the polypeptide comprises an amino acid sequence of ASQLS TAVLG RLSDE LHRLQ DYPRT DVGSG SP-$NH_2$, KCNTATCATQ RLADFLRHSS NNLKPILPPT NVGSNT-trans-Hyp-$NH_2$, or KCNTATCATQ RLAEFLRHSS NNFGPILPPT NVGSNTP-$NH_2$, and preferably, wherein the polypeptide derivative is selected from any one or more of N1, N2, N3, or M1-M30.

**18.** The use of any one of claims 1 and 7-17 or the method of any one of claims 4 and 7-17, wherein the improving the activity selectivity of the polypeptide derivative comprises improving the activity of the polypeptide derivative against an amylin receptor preferentially over a calcitonin receptor, preferably comprises improving a ratio of the activity of the polypeptide derivative against an amylin receptor to the activity of the polypeptide derivative against a calcitonin receptor, and further preferably further comprises improving the activity of the polypeptide derivative against an amylin receptor.

**19.** The use of any one of claims 1 and 7-18 or the method of any one of claims 4 and 7-18, wherein the improving the activity selectivity of the polypeptide derivative comprises improving the ratio of the activity against an amylin receptor to that against a calcitonin receptor in a cell by more than 1.5 folds, preferably more than 2 folds, further preferably more than 5 folds, and still further preferably more than 10 folds.

**20.** The use or the method of claim 18 or claim 19, wherein the activity is agonistic activity.

**21.** The use of any one of claims 1 and 7-20 or the method of any one of claims 4 and 7-20, wherein the polypeptide derivative is an amylin receptor agonist, preferably is an amylin or calcitonin derivative, and more preferably comprises an amino acid sequence of ASQLS TAVLG RLSDE LHRLQ DYPRT DVGSG SP-$NH_2$ or KCNTATCATQ RLADFLRHSS NNLKPILPPT NVGSNT-trans-Hyp-$NH_2$.

**22.** The use of claim 2 or the method of claim 5, wherein the activity of the polypeptide derivative is activity in losing weight and/or reducing food intake.

**23.** The use of any one of claims 2 and 7-17 or the method of any one of claims 5 and 7-17, wherein the improving the activity of the polypeptide derivative is improving the activity of the polypeptide derivative against an amylin receptor and a calcitonin receptor in a cell.

**24.** The use of any one of claims 2, 7-17 and 22-23 or the method of any one of claims 5, 7-17, and 22-23, wherein the polypeptide derivative is an amylin receptor agonist, preferably is an amylin or calcitonin derivative, and more preferably comprises an amino acid sequence of KCNTATCATQ RLADFLRHSS NNLKPILPPT NVGSNT-trans-Hyp-$NH_2$ or KCNTATCATQ RLAEFLRHSS NNFGPILPPT NVGSNTP-$NH_2$.

**25.** The use of any one of claims 3 and 7-17 or the method of any one of claims 6 and 7-17, wherein the polypeptide derivative comprising the fatty acid-containing side chain is co-administered with one or more oral delivery agents, preferably a salt of N-[8-(2-hydroxybenzoyl)amino]caprylic acid (NAC), and more preferably PNAC.

**26.** The use of any one of claims 3 and 7-17 or the method of any one of claims 6 and 7-17, wherein the improving the oral bioavailability of the polypeptide derivative is achieving higher oral bioavailability than a polypeptide molecule of Cagrilintide.

**27.** The use of any one of claims 3 and 7-17 or the method of any one of claims 6 and 7-17, wherein the polypeptide derivative is an amylin receptor agonist, preferably is an amylin or calcitonin derivative, and more preferably comprises an amino acid sequence of KCNTATCATQ RLADFLRHSS NNLKPILPPT NVGSNT-trans-Hyp-$NH_2$ or KCNTATCATQ RLAEFLRHSS NNFGPILPPT NVGSNTP-$NH_2$.

**28.** A derivative of a polypeptide, comprising the polypeptide and a side chain linked thereto in the form of Z1+Z2+Z3, wherein Z1 is a C16-C22 fatty diacid;

Z2 is selected from any one of γGlu, αGlu, βAsp, αAsp, Inp, and Trx, or is absent; and
Z3 is n AEEA(s), wherein $n \geq 0$.

**29.** The derivative of claim 28, wherein Z1 is a C16, C17, C18, C19 or C20 fatty diacid.

**30.** The derivative of claim 28 or 29, wherein Z2 is selected from any one of γGlu, Inp, and Trx, or is absent.

**31.** The derivative of any one of claims 28-30, wherein n is greater than 0.

**32.** The derivative of any one of claims 28-31, wherein Z2 is Inp or Trx; and n is greater than 0.

**33.** The derivative of any one of claims 28-32, wherein Z2 is absent; and n is greater than 0.

**34.** The derivative of any one of claims 28-33, wherein Z2 is yGlu; and n=2.

**35.** The derivative of any one of claims 28-34, wherein the polypeptide is an amylin receptor agonist; preferably, the derivative of the polypeptide is an amylin or calcitonin derivative; and more preferably, the polypeptide comprises an amino acid sequence of ASQLS TAVLG RLSDE LHRLQ DYPRT DVGSG SP-$NH_2$, KCNTATCATQ RLADFLRHSS NNLKPILPPT NVGSNT-trans-Hyp-$NH_2$, or KCNTATCATQ RLAEFLRHSS NNFGPILPPT NVGSNTP-$NH_2$, and preferably, the derivative of the polypeptide is selected from any one or more of N1, N2, N3, or M1-M30.

**36.** A pharmaceutical composition comprising the derivative of any one of claims 28-35 or a pharmaceutically acceptable salt thereof, and a pharmaceutically acceptable excipient, wherein preferably, the composition comprises one or more oral delivery agents, more preferably a salt of N-[8-(2-hydroxybenzoyl)amino]caprylic acid (NAC), and further preferably PNAC.

**37.** Use of the derivative of any one of claims 28-35 or a pharmaceutically acceptable salt thereof or the composition of claim 36 in the manufacture of a polypeptide-based medicament, wherein preferably, the polypeptide-based medicament is used for preventing and/or treating overweight and/or obesity and/or type I or type II diabetes and/or osteoporosis and/or neuropathic pain.

**38.** The use of claim 37, wherein the polypeptide-based medicament is an oral medicament; more preferably, the oral medicament comprises one or more oral delivery agents, preferably a salt of N-[8-(2-hydroxybenzoyl)amino]caprylic acid (NAC), and further preferably PNAC.

**39.** Use of the derivative of any one of claims 28-35 or a pharmaceutically acceptable salt thereof or the pharmaceutical composition of claim 36 in reducing food intake.

**40.** A method of preventing and/or treating overweight and/or obesity and/or type I or type II diabetes and/or osteoporosis and/or neuropathic pain, comprising administering to a subject a prophylactically or therapeutically effective amount of the derivative of any one of claims 28-35 or a pharmaceutically acceptable salt thereof or the pharmaceutical composition of claim 36.

**41.** A method of reducing food intake, comprising administering to a subject an effective amount of the derivative of any one of claims 28-35 or a pharmaceutically acceptable salt thereof or the pharmaceutical composition of claim 36.

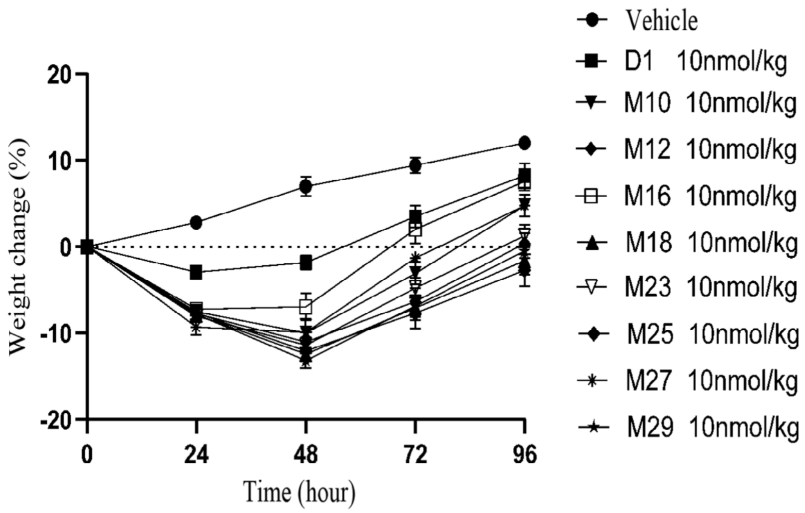


Figure 1

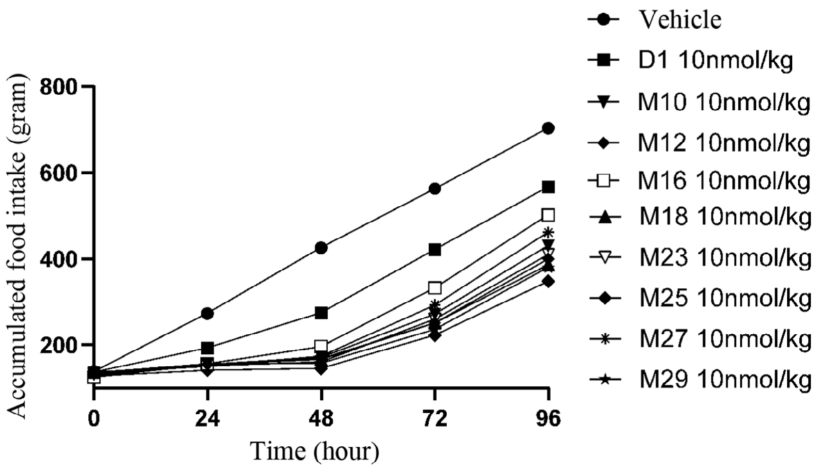


Figure 2

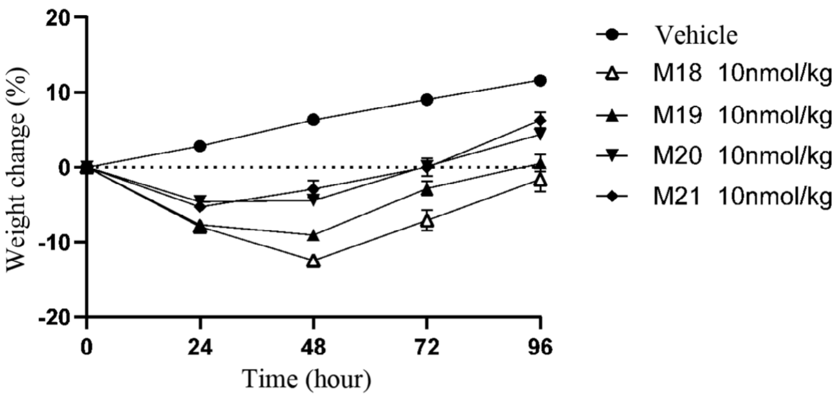


Figure 3

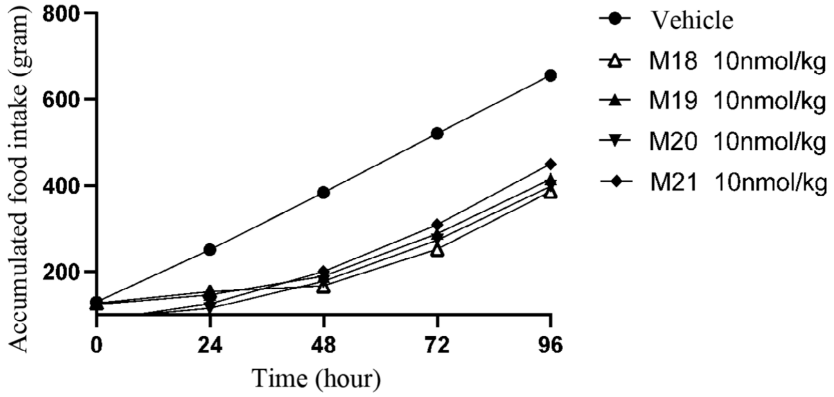


Figure 4

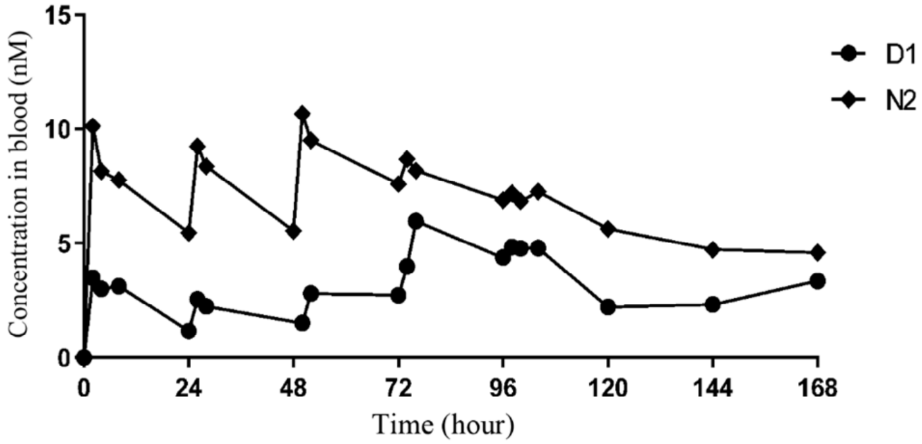


Figure 5

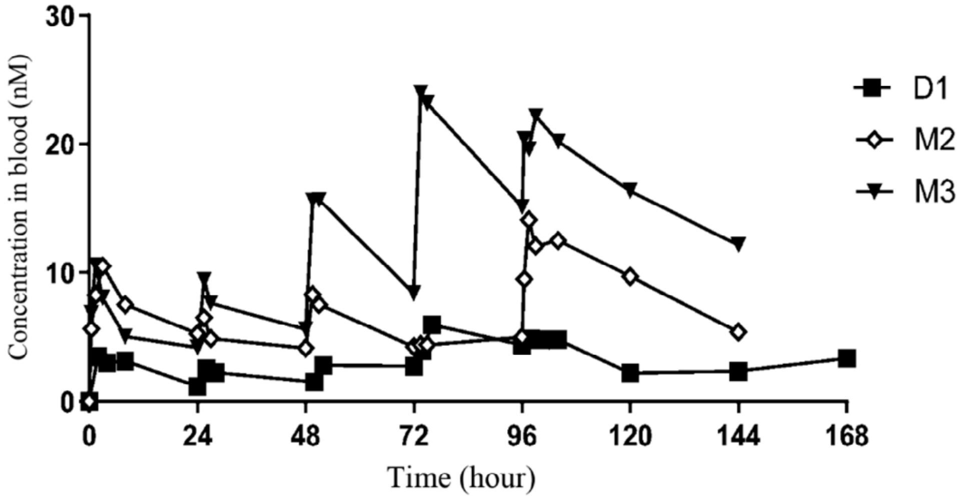


Figure 6

**INTERNATIONAL SEARCH REPORT**

International application No. **PCT/CN2024/095763**

**A. CLASSIFICATION OF SUBJECT MATTER**

A61K38/26(2006.01)i; A61K47/54(2017.01)i; A61K9/00(2006.01)i; A61P3/10(2006.01)i; C07K14/62(2006.01)i

According to International Patent Classification (IPC) or to both national classification and IPC

**B. FIELDS SEARCHED**

Minimum documentation searched (classification system followed by classification symbols)

IPC:A61K,A61P,C07K

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge, 中国专利序列数据库, National Sequence Database of Chinese Patent: 脂肪酸侧链, 修饰, 生物活性, 选择性, 脂肪二酸, AEEA, γGlu, 利用度, 酰胺键, 降钙素受体, 胰淀素受体, 口服递送剂, Fatty Acid Side Chain, Modification, Biological Activity, Selectivity, Fatty Diacid, Availability, Amide Bond, Calcitonin Receptor, Amylin Receptor, Oral Delivery Agents

**C. DOCUMENTS CONSIDERED TO BE RELEVANT**

| Category* | Citation of document, with indication, where appropriate, of the relevant passages | Relevant to claim No. |
|---|---|---|
| X | CN 115947822 A (BEIJING HUIZHIHENG BIOTECHNOLOGY CO., LTD. et al.) 11 April 2023 (2023-04-11)<br>claims 1-15, and description, paragraphs 23-29 | 1-41 |
| X | CN 109485720 A (CHINA PHARMACEUTICAL UNIVERSITY) 19 March 2019 (2019-03-19)<br>claims 1-9, and description, paragraphs 7-31 | 1-41 |
| A | WO 2023028466 A1 (ELI LILLY AND COMPANY) 02 March 2023 (2023-03-02)<br>entire document | 1-41 |
| A | CN 111171134 A (TIANJIN INSTITUTE OF PHARMACEUTICAL RESEARCH CO., LTD.) 19 May 2020 (2020-05-19)<br>entire document | 1-41 |
| A | WO 2019170895 A1 (ENZYPEP B.V.) 12 September 2019 (2019-09-12)<br>entire document | 1-41 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **08 August 2024** | **16 August 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/095763**

**C. DOCUMENTS CONSIDERED TO BE RELEVANT**

| Category* | Citation of document, with indication, where appropriate, of the relevant passages | Relevant to claim No. |
|---|---|---|
| A | CN 113621045 A (TIANJIN INSTITUTE OF PHARMACEUTICAL RESEARCH CO., LTD.) 09 November 2021 (2021-11-09)<br>entire document | 1-41 |
| A | CN 107699588 A (BIOTECHNOLOGY RESEARCH INSTITUTE, CHINESE ACADEMY OF AGRICULTURAL SCIENCES) 16 February 2018 (2018-02-16)<br>entire document | 1-41 |

Form PCT/ISA/210 (second sheet) (July 2022)

**INTERNATIONAL SEARCH REPORT**

International application No.
**PCT/CN2024/095763**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/095763**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **40**
because they relate to subject matter not required to be searched by this Authority, namely:

Claim 40 relates to a method for treatment or diagnosis of a living human or animal body, which falls within the cases set out in PCT Rule 39.1(iv) for which no international search is required. In the present report, a search is carried out on the basis of "the use in the preparation of a drug for preventing and/or treating overweight, obesity and/or type I or type II diabetes and/or osteoporosis and/or neuropathic pain".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/095763**

| Patent document cited in search report | | | Publication date (day/month/year) | Patent family member(s) | | | Publication date (day/month/year) |
|---|---|---|---|---|---|---|---|
| CN | 115947822 | A | 11 April 2023 | None | | | |
| CN | 109485720 | A | 19 March 2019 | WO | 2019047904 | A1 | 14 March 2019 |
| WO | 2023028466 | A1 | 02 March 2023 | KR | 20240038025 | A | 22 March 2024 |
| | | | | TW | 202328055 | A | 16 July 2023 |
| | | | | AU | 2022332278 | A1 | 25 January 2024 |
| | | | | EP | 4392404 | A1 | 03 July 2024 |
| | | | | CA | 3227734 | A1 | 02 March 2023 |
| | | | | IL | 310644 | A | 01 April 2024 |
| CN | 111171134 | A | 19 May 2020 | WO | 2020103729 | A1 | 28 May 2020 |
| | | | | EP | 3882263 | A1 | 22 September 2021 |
| | | | | EP | 3882263 | A4 | 31 August 2022 |
| | | | | JP | 2022507239 | A | 18 January 2022 |
| | | | | JP | 7350851 | B2 | 26 September 2023 |
| | | | | KR | 20210091230 | A | 21 July 2021 |
| WO | 2019170895 | A1 | 12 September 2019 | EP | 3762408 | A1 | 13 January 2021 |
| | | | | US | 2020262886 | A1 | 20 August 2020 |
| | | | | US | 10858414 | B2 | 08 December 2020 |
| CN | 113621045 | A | 09 November 2021 | EP | 4148064 | A1 | 15 March 2023 |
| | | | | EP | 4148064 | A4 | 12 June 2024 |
| | | | | WO | 2021227989 | A1 | 18 November 2021 |
| | | | | KR | 20230008846 | A | 16 January 2023 |
| | | | | JP | 2023525260 | A | 15 June 2023 |
| | | | | US | 2023174608 | A1 | 08 June 2023 |
| CN | 107699588 | A | 16 February 2018 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310614769
- WO 2008028859 A


### Non-patent literature cited in the description


- **FIONA MCCARTNEY et al.** *Journal of Controlled Release*, 28 September 2019, vol. 310, 115-126
- **P. UHL et al.** Nanomedicine: Nanotechnology. *Biology and Medicine*, February 2020, vol. 24, 102132
- **VIVEK GUPTA et al.** *Journal of Controlled Release*, 28 December 2013, vol. 172 (3), 753-762